# EUROPEAN PATENT APPLICATION

(11) **EP 4 424 177 A1**
(43) Date of publication of application: **04.09.2024**
(21) Application number: 22887145.5
(22) Date of filing: 27.10.2022
(51) Int. Cl.: A23L 33/135, A61K 35/744, A61P 31/14

(54) **SARS-COV-2 PROLIFERATION INHIBITOR**

(30) Priority: 29.10.2021 JP 2021177339
(71) Applicant: Japan as Represented by Director-General of National Institute of Infectious Diseases, Tokyo 162-8640 (JP); KIRIN HOLDINGS KABUSHIKI KAISHA, Nakano-ku, Tokyo 164-0001 (JP)
(72) Inventor: ISHII, Hiroshi, Tokyo 162-8640 (JP); MATANO, Tetsuro, Tokyo 162-8640 (JP); JONAI, Kenta, Tokyo 164-0001 (JP); OHSHIO, Konomi, Tokyo 164-0001 (JP); FUJIWARA, Daisuke, Tokyo 164-0001 (JP)
(74) Representative: Hoffmann Eitle
(86) International application number: PCT/JP2022/040242
(87) International publication number: WO 2023/074820

(57) **Abstract**

An object of the present invention is to provide a novel agent for inhibiting proliferation of SARS-CoV-2 and a novel agent for preventing onset of COVID-19. According to the present invention, there is provided an agent for inhibiting proliferation of SARS-CoV-2 comprising a lactic acid bacterium as an active ingredient. Also, according to the present invention, there is provided an agent for preventing onset of COVID-19 comprising a lactic acid bacterium as an active ingredient. The lactic acid bacterium which is the active ingredient of the present invention is preferably *Lactococcus lactis* subsp. *lactis.* The agent of the present invention is preferably in the form of a food composition.

## Description

### CROSS-REFERENCE TO RELATED APPLICATION

The present application enjoys the benefit of priority from the prior Japanese Patent Application No. 2021-177339 filed on October 29, 2021, the entire disclosure of which is incorporated herein by reference.

### TECHNICAL FIELD

The present invention relates to an agent for inhibiting proliferation of SARS-CoV-2 and an agent for preventing onset of COVID-19.

### BACKGROUND ART

Due to global spread of the novel coronavirus (SARS-CoV-2), society's interest in therapeutic drugs, vaccines, diagnostic drugs, and the like for SARS-CoV-2 infectious disease (COVID-19) is increasing. Vaccination has made it possible to avoid an increase in severity after infection, but has not yet completely prevented SARS-CoV-2 infection. Also due to emergence of mutant strains, there are many cases where even vaccinated people have become infected. Therefore, there is an urgent need to develop a preventive drug for SARS-CoV-2. Currently, there are eight drugs, including dexamethasone, which are indicated for SARS-CoV-2 in Japan, and no preventive drugs have been approved to date (Non-Patent Document 1).

### Reference List

### Non-Patent Documents

Non-Patent Document 1: Concept of drug treatment for COVID-19, 13th edition, The Japanese Association for Infectious Diseases, February 10, 2022,
Non-Patent Document 2: Matsuyama S, et al., Proc Natl Acad Sci USA. 117:7001-7003, 2020.
Non-Patent Document 3: Kawase M, et al., J. Virol. 86:6537-6545, 2012.

### SUMMARY OF THE INVENTION

An object of the present invention is to provide a novel agent for inhibiting proliferation of SARS-CoV-2 and a novel agent for preventing onset of COVID-19.

The present inventors have found that a culture supernatant of plasmacytoid dendritic cells (pDC) obtained by being cultured with addition of *Lactococcus lactis* subsp. *lactis,* which is a type of lactic acid bacterium, inhibits proliferation of SARS-CoV-2. The present invention is based on this finding.

According to the present invention, the following inventions are provided.
[1] An agent for inhibiting proliferation of SARS-CoV-2 comprising a lactic acid bacterium as an active ingredient.
[2] The agent according to [1], wherein the lactic acid bacterium is capable of activating plasmacytoid dendritic cells (pDC) and inducing at least interferon (IFN) production.
[3] The agent according to [1] or [2], wherein the lactic acid bacterium is *Lactococcus lactis* subsp. *lactis.*
[4] The agent according to any one of [1] to [3], wherein the lactic acid bacterium is *Lactococcus lactis* subsp. *lactis* JCM 5805.
[5] The agent according to any one of [1] to [4], which is an agent for preventing onset of COVID-19.
[6] The agent according to any one of [1] to [5], which is in the form of a food composition.
[7] The agent according to [6], wherein the food composition is a beverage or supplement.
[8] The agent according to any one of [1] to [5], which is in the form of a pharmaceutical composition.
[9] Use of a lactic acid bacterium, for the manufacture of an agent for inhibiting proliferation of SARS-CoV-2, an agent for preventing onset of COVID-19 or an agent for relieving a symptom of COVID-19, as an agent for inhibiting proliferation of SARS-CoV-2, an agent for preventing onset of COVID-19 or an agent for relieving a symptom of COVID-19, or in a method for inhibiting proliferation of SARS-CoV-2, a method for preventing onset of COVID-19 or a method for relieving a symptom of COVID-19.
[10] A lactic acid bacterium or a composition comprising the lactic acid bacterium, for use in inhibiting proliferation of SARS-CoV-2, preventing onset of COVID-19 or relieving a symptom of COVID-19.
[11] A method for inhibiting proliferation of SARS-CoV-2, a method for preventing onset of COVID-19 or a method for relieving a symptom of COVID-19, which method comprises a step of feeding or administering an effective amount of a lactic acid bacterium or a composition comprising the lactic acid bacterium to a subject in need thereof.

The lactic acid bacterium, which is the active ingredient of the present invention, is one of food materials that have been safely eaten along with fermented foods for a long time. Therefore, the agent of the present invention is advantageous in that it can be utilized as a functional food or medicine that imparts an effect for inhibiting proliferation of SARS-CoV-2 or an effect for preventing onset of COVID-19, and can also be used as a functional food or medicine that is safe for mammals including humans. In particular, the agent of the present invention is advantageous in that it can be ingested daily as a food and can easily inhibit proliferation of SARS-CoV-2 and prevent onset of COVID-19.

### BRIEF DESCRIPTION OF THE DRAWINGS

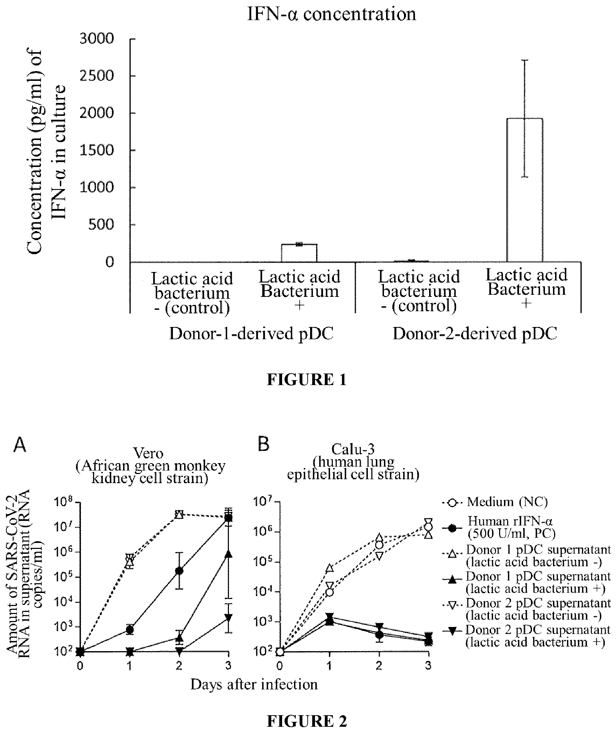

FIG. 1 shows the concentration (pg/ml) of interferon-α (IFN-α) in a culture supernatant when a lactic acid bacterium was added to plasmacytoid dendritic cells (pDC) derived from Donor 1 or Donor 2. The lactic acid bacterium - and the lactic acid bacterium + in this figure represent a culture supernatant obtained without addition of the lactic acid bacterium to the pDC and a culture supernatant obtained with addition of the lactic acid bacterium to the pDC, respectively.
FIG. 2 shows a change in amount of RNA of the SARS-CoV-2 virus when Vero cells (FIG. 2A) or Calu-3 cells (FIG. 2B) were infected with the virus. The vertical axis indicates the amount of RNA of the SARS-CoV-2 virus in a culture supernatant, and the horizontal axis indicates the number of days after infection. The NC and PC in the figure represent a negative control and a positive control, respectively. The lactic acid bacterium - and the lactic acid bacterium + in this figure represent a culture supernatant obtained without addition of the lactic acid bacterium to the pDC, and a culture supernatant obtained with addition of the lactic acid bacterium to the pDC, respectively.

### DETAILED DESCRIPTION OF THE INVENTION

The agent for inhibiting proliferation of SARS-CoV-2 and the agent for preventing onset of COVID-19 of the present invention (hereinafter, the two agents are collectively referred to as "the agent of the present invention" in some cases) each comprise a lactic acid bacterium as an active ingredient.

A lactic acid bacterium which is capable of activating plasmacytoid dendritic cells (pDC) and inducing at least interferon (IFN) production can be used as the lactic acid bacterium, which is the active ingredient of the present invention. Preferably, there can be used a lactic acid bacterium having the nature of inducing production of 50 pg/mL or more, preferably 100 pg/mL or more of IFN-α when added at 10 µg/mL to human peripheral blood mononuclear cell-derived plasmacytoid dendritic cells (pDC) culture.

The lactic acid bacterium of the present invention is capable of inducing all of IFNs, i.e., Type I IFN (type I interferon), Type II IFN (type II interferon), and Type III IFN (type III interferon). Type I IFN refers to cytokines that are regarded as being effective against viral infection, including, for example, IFN-α (1, 2, 4, 5, 6, 7, 8, 10, 13, 14, 16, 17 and 21) and IFN-β. Type II IFN includes IFN-γ, and Type III IFN includes IFN-λ. Among the lactic acid bacteria of the present invention, those having an activity of inducing production of Type I IFN are particularly preferred. Upon activation of pDC by the lactic acid bacterium of the present invention, a cell protrusion, which is a characteristic of activated dendritic cells, appears, and the production of Type I IFN and Type III IFN is induced. Further, the production of Type II IFN such as IFN-γ from NK cells and Th1 cells can also be induced.

The IFN that can be induced to be produced by the lactic acid bacterium which is the active ingredient of the present invention is not particularly limited, but is preferably one or more selected from the group consisting of IFN-α, IFN-β, and IFN-λ, more preferably two or more selected from the group consisting of IFN-α, IFN-β, and IFN-λ, further preferably two or more selected from the group consisting of IFN-α, IFN-β, and IFN-λ, at least one of the two or more IFNs being IFN-α, and particularly preferably two or more selected from the group consisting of IFN-α, IFN-β, and IFN-λ, at least two of the two or more IFNs being IFN-α and IFN-β.

Whether the lactic acid bacterium, which is the active ingredient of the present invention, is capable of activating pDC and inducing at least interferon (IFN) production can be determined, for example, by measuring whether the pDC are activated and IFN production is induced when candidate lactic acid bacteria are cultured in the coexistence of bone marrow cells of mammals such as mice. For example, for the IFN-α concentration, the concentration of IFN-α in a culture solution may be measured by ELISA or the like. Specifically, mouse bone marrow cells from which red blood cells had been removed are suspended in an RPMI medium (manufactured by SIGMA) containing 10% FCS and 2 µM β-mercaptoethanol so as to attain a concentration of 5 × 10⁵ cells/mL; Flt-3L is added as a pDC-inducing cytokine to the resulting cell suspension at a final concentration of 100 ng/ml; the mixture is cultured in a CO₂ incubator at 37°C and 5% CO₂; after 7 days, a lactic acid bacterial strain is added at 10 µg/ml; after 48 hours, a culture supernatant is recovered; and the concentration of IFN-α in the culture supernatant can be measured by ELISA using an IFN-α measurement kit (PBL).

The lactic acid bacterium which is the active ingredient of the present invention is not particularly limited, and examples thereof include lactic acid-producing cocci, such as lactic acid bacteria belonging to the genus *Lactococcus,* the genus *Leuconostoc,* the genus *Pediococcus,* the genus *Streptococcus* and the genus *Enterococcus.* The lactic acid bacterium which is the active ingredient of the present invention is preferably a lactic acid bacterium belonging to the genus *Lactococcus* or *Pediococcus.*

Examples of the bacteria belonging to the genus *Lactococcus* include *Lactococcus lactis, Lactococcus lactis* subsp. *lactis, Lactococcus garvieae, Lactococcus lactis* subsp. *cremoris,* and *Lactococcus lactis* subsp. *hordniae. Lactococcus lactis* subsp. *lactis* is preferred from the viewpoint that it is capable of activating pDC and inducing at least interferon (IFN) production.

Specific examples of the bacteria belonging to the genus *Lactococcus* include *Lactococcus lactis* subsp. *lactis* JCM 5805, *Lactococcus lactis* subsp. *lactis* NBRC 12007, *Lactococcus lactis* subsp. *lactis* NRIC 1150, *Lactococcus lactis* subsp. *lactis* JCM 20101, *Lactococcus lactis* subsp. *lactis* JCM 7638, *Lactococcus lactis* subsp. *lactis* ATCC 11454, *Lactococcus garvieae* NBRC 100934, *Lactococcus lactis* subsp. *cremoris* JCM 16167, *Lactococcus lactis* subsp. *cremoris* NBRC 100676, *Lactococcus lactis* subsp. *hordniae* JCM 1180 and *Lactococcus lactis* subsp. *hordniae* JCM 11040. *Lactococcus lactis* subsp. *lactis* JCM 5805 is preferred from the viewpoint that it is capable of activating pDC and inducing at least interferon (IFN) production.

Examples of bacteria belonging to the genus *Leuconostoc* include *Leuconostoc lactis.* Specific examples of the bacteria belonging to the genus *Leuconostoc* include *Leuconostoc lactis* NBRC 12455.

Examples of bacteria belonging to the genus *Pediococcus* include *Pediococcus acidilactici, Pediococcus pentosaceus, Pediococcus cellicola, Pediococcus claussenii*, *Pediococcus damnosus, Pediococcus ethanolidurans*, *Pediococcus inopinatus, Pediococcus parvulus,* and *Pediococcus stilesii.* Specific examples of the bacteria belonging to the genus *Pediococcus* include *Pediococcus acidilactici* JCM 8797 and *Pediococcus damnosus* JCM 5886.

Examples of bacteria belonging to the genus *Streptococcus* include *Streptococcus* thermophilus.

In the present invention, the lactic acid bacteria are available from known depositary institutions and the like. For example, among the above lactic acid-producing coccal strains, the JCM bacterial strains are available from Microbe Division, RIKEN BioResource Research Center (3-1-1 Koyadai, Tsukuba, Ibaraki); the NBRC bacterial strains are available from Biological Resource Center, National Institute of Technology and Evaluation (NBRC) (2-5-8 Kazusa-Kamatari, Kisarazu, Chiba); the NRIC bacterial strains are available from NODAI Culture Collection Center, Tokyo University of Agriculture (1-1-1 Sakuragaoka, Setagaya-ku, Tokyo); and the ATCC bacterial strains are available from American Type Culture Collection (U.S.A.).

The lactic acid bacterium used as the active ingredient in the present invention includes a culture of a lactic acid bacterium. The culture includes, for example, live bacterial cells, dead bacterial cells, crushed products of live bacterial cells and dead bacterial cells, lyophilized products of live bacterial cells and dead bacterial cells, crushed products of the lyophilized products, enzymatically treated products of live bacterial cells and dead bacterial cells, culture solutions and culture solution extracts, and also includes parts of lactic acid bacteria and treated products of lactic acid bacteria. The dead bacterial cells can be obtained, for example, by heating treatment, treatment with a drug such as an antibiotic, treatment with a chemical such as formalin, treatment with ultraviolet rays, and treatment with radiation such as γ rays. Further, the DNAs or RNAs of the lactic acid bacteria described above are also included in the culture of the lactic acid bacterium. It is considered that the DNAs or RNAs of the lactic acid bacteria are capable of activating pDC and inducing IFN production. The treated product includes, for example, heated bacterial cells (dead bacterial cells), lyophilized products thereof, and cultures containing these, and further includes a solution of bacteria crushed by ultrasonic waves or the like, and a solution of enzymatically treated bacteria. The treated product also includes a treated product in which cell walls have been removed by an enzyme or mechanical means. Further, the treated product also includes a nucleic acid-containing fraction obtained by dissolving bacteria with a surfactant or the like and then subjecting them to precipitation with ethanol or the like. Furthermore, the bacterial cells may also include dead bacterial cells.

The lactic acid bacterium can be cultivated by a known method using a known medium. The medium can be MRS medium, GAM medium, or LM17, and may be added with an inorganic salt, a vitamin, an amino acid, an antibiotic, serum, or the like as appropriate and used. The culturing may be carried out at 25 to 40°C for several hours to several days.

After the cultivation, the lactic acid bacterial cells are collected by centrifugation or filtration. When used as dead bacteria, the bacterial cells may be killed and inactivated using an autoclave or the like.

The lactic acid bacterium, which is the active ingredient of the present invention, is preferably fed orally from the viewpoint of alleviating a burden of feeding for obtaining the effect for inhibiting proliferation of SARS-CoV-2 and the effect for preventing onset of COVID-19 on a living body. Such a lactic acid bacterium preferably has high resistance to gastric juices and intestinal juices, for example, strong acid resistance. The lactic acid bacterium is not particularly limited, and can be either a live bacterium or a dead bacterium. However, from the viewpoints of the effect for inhibiting proliferation of SARS-CoV-2, the effect for preventing onset of COVID-19, and production efficiency, the lactic acid bacterium is preferably a dead bacterium.

Here, the lactic acid bacterium which is the active ingredient of the present invention is capable of activating pDC and inducing at least IFN production, and is not intended to directly administer IFN to a living body. Direct administration of IFN to a living body can include oral administration and parenteral administration. When IFN is directly administered orally to a living body, IFN is decomposed due to its low resistance to gastric juices and intestinal fluids, and is unlikely to be absorbed into the living body as it is. Further, the direct parenteral administration of IFN to a living body can include, for example, subcutaneous administration. However, the subcutaneous administration has been reported to bring about a high feeding burden and the possibility of side effects (fever, headache, depression, and the like). Thus, it can be said that the lactic acid bacterium, which is the active ingredient of the present invention, is superior to the direct administration of IFN to a living body, in that the lactic acid bacterium can be fed orally so that an administration burden is alleviated; in that the possibility of side effects can be reduced; and in the effect for inhibiting proliferation of SARS-CoV-2 and the effect for preventing onset of COVID-19.

The agent of the present invention can be provided in the form of a composition comprising the lactic acid bacterium. In this case, the agent of the present invention can be provided by mixing the lactic acid bacterium with other components (e.g., a food raw material, a food additive, a raw material for a supplement, and a formulation additive). The content of the lactic acid bacterium in the composition can be determined based on the manner of providing the agent and the effective ingestion amount which will be described below. A lower limit value (or more or more than) of the content can be, for example, 0.01% by mass, 0.1% by mass, 1% by mass, 10% by mass, or 20% by mass, and an upper limit value (or less or less than) thereof can be, for example, 100% by mass, 90% by mass, 80% by mass, 70% by mass, 60% by mass, or 50% by mass or less. The lower and upper limit values can be combined arbitrarily, and the content of the lactic acid bacterium in the composition can be, for example, 0.01 to 90% by mass or 0.1 to 50% by mass.

According to the present invention, a composition for inhibiting proliferation of SARS-CoV-2, a composition for preventing onset of COVID-19 and a composition for alleviating a symptom of COVID-19, each comprising the lactic acid bacterium, are provided. The composition of the present invention can be implemented according to the descriptions regarding the agent of the present invention.

As will be described in the Examples below, a culture supernatant of plasmacytoid dendritic cells (pDC) obtained by being cultured with addition of *Lactococcus lactis* subsp. *lactis,* which is a type of lactic acid bacterium, exhibited the effect for inhibiting proliferation of SARS-CoV-2. This effect was confirmed in SARS-CoV-2-sensitive cells pretreated with the pDC culture supernatant. Therefore, in the present invention, the lactic acid bacterium can be used as an active ingredient for inhibiting proliferation of SARS-CoV-2, and can also be used as an active ingredient for preventing onset of COVID-19.

*Lactococcus lactis* subsp. *lactis,* which is a type of lactic acid bacterium, can exert an action of inducing INF-α production on a living body even when fed orally (JP 2017-201984 A). Therefore, oral feeding of the lactic acid bacterium is considered to provide an anti-SARS-CoV-2 action including induction of INF-α production *in vivo,* and provides an action of inhibiting proliferation of SARS-CoV-2 and an action of preventing onset of COVID-19.

SARS-CoV-2 includes not only the first discovered viral strain, but also mutant strains thereof (for example, B.1.1.7 lineage (alpha strain), B.1.351 lineage (beta strain), P.1 lineage (gamma strain), B.1.617.2 lineage (delta strain), and B.1.1.529 lineage (omicron strain)). SARS-CoV-2 is synonymous with severe acute respiratory syndrome coronavirus-2, and the SARS-CoV-2 infectious disease (diseases and symptoms caused by infection with SARS-CoV-2) is referred to as COVID-19 (novel coronavirus infection).

The agent of the present invention can be provided in the form of a food, a medicine, a quasi-drug, a feed (including a pet food), an additive, or the like, and can be implemented according to the following descriptions.

The active ingredient of the present invention can be orally fed to a human and a non-human animal, and a typical form of feeding is a food (e.g., a food composition). When the active ingredient of the present invention is provided as a food, the active ingredient can be provided as it is as a food, or can be provided by being incorporated in a food. The food thus provided is a food containing an effective amount of the active ingredient of the present invention. As used herein, the phrase "containing an effective amount" of the active ingredient of the present invention refers to a content of the active ingredient of the present invention to be ingested, within the range as will be described below, when an individual food is ingested in a normally-eaten amount. The meaning of the "food" as used herein includes health foods, functional foods, nutritional supplements, foods with health claims (such as foods for specified health uses, foods with nutrient function claims, and foods with function claims), foods for special dietary uses (such as foods for infants, foods for expectant and nursing mothers, and foods for sick persons) and supplements. When the active ingredient of the present invention is fed to an animal other than a human, needless to say, the food referred to herein is used as a feed.

The active ingredient of the present invention has the effect for inhibiting proliferation of SARS-CoV-2 and the effect for preventing onset of COVID-19 as described above, and thus can be provided by being incorporated in a food ingested daily. In this case, the agent of the present invention can be provided in a unit package form in which the ingestion amount per meal is predetermined. Examples of the unit package form per meal include forms which define a constant amount using a pack, a package, a can, a bottle and the like. In order to allow the agent of the present invention to exert various actions better, the ingestion amount per meal can be determined according to the daily ingestion amount of the active ingredient of the present invention which will be described later. The food of the present invention may be provided in the form of a package on which an explanation about the ingestion amount is given, or may be provided together with a document or the like which explains the ingestion amount.

The predetermined ingestion amount per meal in the unit package form may be either the effective daily ingestion amount or an ingestion amount obtained by dividing the effective daily ingestion amount into two or more (preferably two or three) portions. Thus, the unit package form of the agent of the present invention can contain the active ingredient of the present invention in the daily ingestion amount for a human which will be described later, or can contain the active ingredient of the present invention in an amount half to one sixth of the daily ingestion amount for a human which will be described later. For convenience of ingestion, the agent of the present invention is preferably provided in the unit package form per meal (i.e., the unit package form per day) in which the ingestion amount per meal is the effective daily ingestion amount.

The form of the "food" is not particularly limited, and the food may be provided, for example, in a beverage form, in a semi-liquid or gelled form, or in a solid or powder form. The "supplement" includes tablets manufactured by adding an excipient, a binder and the like to the active ingredient of the present invention, kneading them together and then tableting the kneaded product, granules manufactured by adding an excipient, a binder and the like to the active ingredient of the present invention and granulating them, orally disintegrating tablets, and capsule agents in which the active ingredient of the present invention is encapsulated in a capsule and the like. When the agent of the present invention is provided as a supplement, it is also suitable to provide it not only in the above-described unit package form per meal or per day, but also in a unit package form per week, per two weeks, per month, or per two months. In the case of the latter unit package form, for example, it is desirable that the ingestion amount per meal or per day should be indicated so that the person who ingests the supplement himself/herself can ingest the effective amount of the active ingredient of the present invention according to the indication.

The food provided in the present invention is not particularly limited as long as it contains the active ingredient of the present invention, and examples thereof can include non-alcoholic beverages such as refreshing drinks, carbonated drinks, drinks containing fruit juice, drinks containing vegetable juice, drinks containing fruit juice and vegetable juice, animal milk such as cow milk, soybean milk, milk beverages, drink-type yogurt, drink-type and stick-type jellies, coffee, cocoa, tea drinks, nutritional drinks, energy drinks, sports drinks, mineral water, flavored water, and non-alcohol beer-taste beverages; carbohydrate-containing foods and beverages such as rice, noodles, bread and pasta; dairy products such as cheese, hard-type or soft-type yogurt, fresh cream composed of animal milk and other oil and fat raw materials, and ice cream; various confectioneries such as Western-style confectioneries including cookies, cakes and chocolate, Japanese-style confectioneries including buns with a bean-jam filling and sweet jellies of adzuki beans, tablet confectioneries (refreshing confectioneries) including soda pop-flavored sweets, candies, gums, gummies, chilled sweets and frozen sweets including jellies and puddings, and snacks; alcoholic beverages such as whiskey, bourbon, spirit, liqueur, wine, fruit wine, sake (Japanese rice wine), Chinese liquor, shochu (Japanese distilled spirit), beer, non-alcohol beer having an alcohol content of 1% or less, low-malt beer, other miscellaneous liquors and shochu mixed with soda water; processed products in which eggs are used, processed products of fish and meat (including giblets such as lever) (including rare delicacy), processed foods such as soup including miso soup, flavorings such as miso, soy sauce, rice seasoning and other seasonings, and liquid diets such as high density liquid diets. It should be noted that mineral water includes both of effervescent mineral water and non-effervescent mineral water.

Tea drinks include all of fermented tea, semi-fermented tea and unfermented tea, and examples thereof include black tea, green tea, barley tea, genmaicha (brown rice tea), sencha (middle-grade green tea), gyokurocha (refined green tea), hojicha (roasted green tea), oolong tea, turmeric herbal tea, Pu-erh tea, rooibos tea, rose tea, chrysanthemum tea, ginkgo leaf tea and herb tea (such as mint tea and jasmine tea).

Examples of fruits used in drinks containing fruit juice and drinks containing fruit juice and vegetable juice include apple, orange, grape, banana, pear, peach, mango, acai, blueberry and ume (plum). Examples of vegetables used in drinks containing vegetable juice and drinks containing fruit juice and vegetable juice include tomato, carrot, celery, pumpkin, cucumber and watermelon.

When the active ingredient of the present invention is provided as a medicine, quasi-drug, or pharmaceutical composition, it can be provided by being formulated in an oral or parenteral agent. The oral agent includes granules, powders, tablets (including sugar-coated tablets), pills, capsule agents, syrups, liquid preparations, jellies, emulsions and suspensions. The parenteral agent includes injections suitable for local administration (including intradermal injection, subcutaneous injection, intramuscular injection, and intravenous injection), inhalants (e.g., inhalation aerosols, inhalation powder preparations, and inhalation liquid preparations), nasal preparations (e.g., nasal powder preparations and nasal liquid preparations), ointments, creams, gels, suppositories, patches, and poultices. These formulations can be formulated using a pharmaceutically acceptable carrier by a technique commonly carried out in the art. The pharmaceutically acceptable carrier includes excipients, binders, diluents, additives, perfumes, buffers, thickeners, colorants, stabilizers, emulsifiers, dispersants, suspending agents, and preservatives.

With regard to the mechanism of SARS-CoV-2 infection, it is known that the virus binds to and adsorbs onto the ACE2 (angiotensin converting enzyme 2) receptor on surfaces of cells, and enters the cells through the binding and adsorption, leading to infection. Therefore, the active ingredient of the present invention is locally administered to a tissue in which the ACE receptor is highly expressed, and thus can provide the effect for inhibiting proliferation of SARS-CoV-2, the effect for preventing onset of COVID-19, and the effect for relieving a symptom of COVID-19 better. Thus, the medicine and quasi-drug of the present invention can be formulated as formulations intended to be administered to the tissue in which the ACE receptor is highly expressed. Examples of the tissue in which the ACE2 receptor is highly expressed include respiratory organs (e.g., nasal cavity, pharynx, larynx, trachea, bronchi, and lungs), and thus the medicine and quasi-drug of the present invention can be formulated as formulations (e.g., inhalants, aerosols and nasal preparations) intended to be administered to the respiratory organs.

When the active ingredient of the present invention is provided as a food, medicine or quasi-drug, it can also be utilized as an agent for relieving a symptom of COVID-19. Namely, the active ingredient of the present invention is taken before or after infection with SARS-CoV-2 (preferably at an initial stage after infection, more preferably within 5 days after infection), and thus the symptom of the infectious disease (COVID-19) is expected to be relieved (especially, the increase in severity of the infectious disease is expected to be relieved).

When the active ingredient of the present invention is provided as a feed, the active ingredient can be implemented according to the above descriptions regarding foods.

When the active ingredient of the present invention is provided as an additive, the active ingredient can be implemented according to the above descriptions regarding foods, medicines and quasi-drugs. The active ingredient of the present invention is provided as a food additive, the active ingredient of the present invention can be used as a functional component having the effect for inhibiting proliferation of SARS-CoV-2 or the effect for preventing onset of COVID-19.

The ingestion amount of the active ingredient of the present invention can be determined depending on the recipient's sex, age and weight, symptoms, ingestion time, dosage form, ingestion route and drugs to be combined. The daily ingestion amount for an adult of the active ingredient of the present invention can be specified, for example, by the number of bacteria. A lower limit value (or more or more than) of the ingestion amount can be 1 × 10⁸ cells, 1 × 10⁹ cells, or 1 × 10¹⁰ cells, and an upper limit value (or less or less than) thereof can be 1 × 10¹⁴ cells, 1 × 10¹³ cells, or 1 × 10¹² cells. The upper and lower limit values can be combined arbitrarily, and the range of the injection amount can be, for example, 1 × 10⁸ to 1 × 10¹⁴ cells, 1 × 10⁹ to 1 × 10¹³ cells, or 1 × 10¹⁰ to 1 × 10¹² cells. The number of lactic acid bacteria can be measured, for example, by fluorescent staining, flow cytometry, and cultivation.

The daily ingestion amount for an adult of the active ingredient of the present invention can also be specified by the dry cell mass. A lower limit value (or more or more than) of the ingestion amount can be 1 mg, 10 mg or 25 mg, and an upper limit value (or less or less than) thereof can be 1000 mg, 500 mg or 100 mg. The upper and lower limit values can be combined arbitrarily, and the range of the injection amount can be, for example, 1 to 1000 mg, 10 to 500 mg, or 25 to 100 mg.

The above-described ingestion amount of, and the below-described ingestion timing and ingestion period for the active ingredient of the present invention are applicable when the active ingredient of the present invention is used for both non-therapeutic and therapeutic purposes, and the ingestion can be read as administration in the case of therapeutic purposes. The active ingredient of the present invention can also be fed to mammals other than humans (e.g., cows, horses, sheep, pigs, dogs and cats). The ingestion amount, ingestion timing and ingestion period can be determined with reference to the descriptions regarding humans.

The active ingredient of the present invention is preferably ingested continuously during a period when the effect for inhibiting proliferation of SARS-CoV-2 or the effect for preventing onset of COVID-19 is expected. The ingestion period for the active ingredient of the present invention can be set to one week or longer, two weeks or longer, or three weeks or longer, preferably one month or longer (four weeks or longer), from the viewpoint of providing the effect for inhibiting proliferation of SARS-CoV-2 or the effect for preventing onset of COVID-19 better. The ingestion interval for the active ingredient of the present invention can be set to once every three days, once every two days or once a day, and is preferably once a day.

The ingestion of the active ingredient of the present invention may also be started before an event or time at which the effect for inhibiting proliferation of SARS-CoV-2 or the effect for preventing onset of COVID-19 is expected. Examples of the event at which the effect for inhibiting proliferation of SARS-CoV-2 or the effect for preventing onset of COVID-19 is expected include actions that may lead to infection with SARS-CoV-2 (e.g., participation in affairs with a high risk of infection, travel to epidemic areas). Examples of the time at which the effect for inhibiting proliferation of SARS-CoV-2 or the effect for preventing onset of COVID-19 is expected include epidemic periods of SARS-CoV-2. Examples of the ingestion timing before the event at which the effect for inhibiting proliferation of SARS-CoV-2 or the effect for preventing onset of COVID-19 is expected include one day or longer, three days or longer, one week or longer, two weeks or longer, three weeks or longer, one month or longer (four weeks or longer), or two months or longer (eight weeks or longer) before the event. In addition, without any particular limitation, the active ingredient of the present invention can also be ingested continuously at ingestion intervals, depending on the case, in a period from the start of ingestion until the event at which the effect for inhibiting proliferation of SARS-CoV-2 or the effect for preventing onset of COVID-19 is expected. The ingestion of the active ingredient of the present invention may also be started after an event or time at which the effect for inhibiting proliferation of SARS-CoV-2 or the effect for preventing onset of COVID-19 is expected. Examples of the ingestion timing after the event at which the effect for inhibiting proliferation of SARS-CoV-2 or the effect for preventing onset of COVID-19 is expected include one day or longer, three days or longer, one week or longer, and two weeks or longer after the event. In addition, without any particular limitation, the active ingredient of the present invention can also be ingested continuously at ingestion intervals, depending on the case, when ingested after the event at which the effect for inhibiting proliferation of SARS-CoV-2 or the effect for preventing onset of COVID-19 is expected. In the present invention, particularly preferably, the ingestion of the active ingredient of the present invention can be started before the event at which the effect for inhibiting proliferation of SARS-CoV-2 or the effect for preventing onset of COVID-19 is expected, and continued until after the event.

According to another aspect of the present invention, there is provided a method for inhibiting proliferation of SARS-CoV-2, a method for preventing onset of COVID-19 or a method for relieving a symptom of COVID-19, the method comprising feeding or administering an effective amount of a lactic acid bacterium or a composition comprising the lactic acid bacterium to a subject in need thereof. The method of the present invention can be implemented according to the descriptions regarding the agent of the present invention.

According to still another aspect of the present invention, there is provided use of a lactic acid bacterium, for the manufacture of an agent for inhibiting proliferation of SARS-CoV-2, an agent for preventing onset of COVID-19 or an agent for relieving a symptom of COVID-19, as an agent for inhibiting proliferation of SARS-CoV-2, an agent for preventing onset of COVID-19 or an agent for relieving a symptom of COVID-19, or in a method for inhibiting proliferation of SARS-CoV-2, a method for preventing onset of COVID-19 or a method for relieving a symptom of COVID-19. The use of the present invention can be implemented according to the descriptions regarding the agent of the present invention and the method of the present invention.

According to further still another aspect of the present invention, there is provided a lactic acid bacterium for use in inhibiting proliferation of SARS-CoV-2, preventing onset of COVID-19 or relieving a symptom of COVID-19. The lactic acid bacterium described above can be implemented according to the descriptions regarding the agent of the present invention.

The method of the present invention and the use of the present invention may be uses in mammals including humans, and are intended to involve both of therapeutic use and non-therapeutic use. The "non-therapeutic," as used herein, means elimination of an activity of operating on, treating, or diagnosing a human (i.e., medical activity to a human), and specifically means elimination of a method of performing operation on, treatment of, or diagnosis involving a human by a doctor or a person who receives an instruction from the doctor.

### EXAMPLES

Hereinafter, the present invention will be described in more detail by way of the following examples, but is not limited to these examples.

### Example 1: Evaluation of effect for inhibiting proliferation of SARS-CoV-2

In Example 1, the effect for inhibiting proliferation of SARS-CoV-2 by a culture supernatant of plasmacytoid dendritic cells (pDC) added with a lactic acid bacterium was evaluated.

### (1) Method

### a. Separation of plasmacytoid dendritic cells (pDC)

The magnetic bead method using EasySep Human Plasmacytoid DC Isolation Kit (manufactured by STEMCELL Technologies) was used to separate plasmacytoid dendritic cells (pDC) from Donor 1- and Donor 2-derived human peripheral blood nuclear cells (hPBMC) (manufactured by iQ Biosciences).

### b. Condition for culturing pDC

The conditions for culturing the pDC (7×10⁴ cells) separated in the above a are as follows. An RPMI-1640 medium (manufactured by Sigma-Aldrich Co. LLC) containing 10% FCS and 1% penicillin-streptomycin was used as the medium, and the pDC were seeded in a 96-well round-bottom plate (manufactured by Corning Incorporated). Then, heat-killed bacterial cells of the *Lactococcus lactis* subsp. *lactis* JCM 5805 strain as the lactic acid bacterium were adjusted with PBS (manufactured by Takara Bio Inc.) to attain a concentration of 1 mg/ml, and added to the medium to attain a final concentration of 10 µg/ml (no lactic acid bacterium was added to a control) and cultured at 37°C for 48 hours in a CO₂ incubator.

### c. Measurement of interferon-α

The concentration of interferon-α (IFN-α) in the culture supernatant obtained by culturing the pDC in the above b was measured using Human IFN-α ELISA Kit (manufactured by PBL Biochemical Laboratories).

### d. Quantification of RNA of SARS-CoV-2

In a 96-well flat-bottom plate (manufactured by Corning Incorporated), 1×10⁴ Vero cells (African green monkey kidney cell strain, see Non-Patent Document 2, JCRB1819 VeroE6/TMPRSS2 (JCRB Cell Bank) was available) or Calu-3 cells (human lung epithelial cell strain, see Non-Patent Document 3, HTB-55^{™} (ATCC) was available) were seeded. After 6 hours, the pDC culture supernatant in the above b (without addition of the lactic acid bacterium (control) or with addition of the lactic acid bacterium) was added to the medium, and pretreated at 37°C for 18 hours in the CO₂ incubator. The pretreated cells were washed with PBS and infected with SARS-CoV-2 (wk-521 strain (National Institute of Infectious Diseases), see Non-Patent Document 2) with TCID₅₀ of 1×10² (Vero, MOI=0.01) or TCID₅₀ of 1×10⁴ (Calu-3, MOI=1). After 1 hour, the cells were washed with PBS, and RNA of the SARS-CoV-2 virus in the culture supernatant 1 to 3 days after infection was extracted using QIAamp Viral RNA Mini Kit (manufactured by QIAGEN). Then, the extracted RNA was quantified by the real-time PCR method. In the real-time PCR analysis, the reaction was made using QuantiTect Probe RT-PCR Kit (manufactured by QIAGEN) and a gene-specific SARS-CoV-2 primer, according to the general reaction composition, 50°C for 30 minutes, 95°C for 15 seconds, then in 45 cycles of 95°C for 15 seconds and 60°C for 60 seconds. The positive control used was cells pretreated, in the same manner, in a medium containing 500 U/ml (equivalent to 2 ng/ml) of human recombinant interferon alpha (rIFN-α).

### (2) Result

The results were as shown in FIGS. 1 and 2. In FIG. 1, it was confirmed that, for both of the Donor 1- and Donor 2-derived pDC, the pDC with addition of the lactic acid bacterium (lactic acid bacterium +) showed high concentration of interferon-α (IFN-α) in the culture supernatant as compared with the pDC without addition of the lactic acid bacterium (lactic acid bacterium -) (control). In FIG. 2, both of the Vero cells (FIG. 2A) and Calu-3 cells (FIG. 2B) pretreated with the pDC culture supernatant obtained without addition of the lactic acid bacterium (lactic acid bacterium -) showed an amount of RNA of the SARS-CoV-2 virus equivalent to that when the medium alone was added (negative control (NC)). On the other hand, the Vero cells and Calu-3 cells pretreated with the pDC culture supernatant obtained with addition of the lactic acid bacterium (lactic acid bacterium +) showed a remarkably small amount of RNA of the SARS-CoV-2 virus as compared with those of the negative control group and the lactic acid bacterium-free group. It was confirmed that, especially, the Vero cells pretreated with the Donor 1- or Donor 2-derived pDC culture supernatant (with addition of the lactic acid bacterium (lactic acid bacterium +)) showed a small amount of RNA of the SARS-CoV-2 virus as compared with the Vero cells pretreated with rIFN-α (500 U/ml) (positive control (PC)) (FIG. 2A).

## Claims

1. An agent for inhibiting proliferation of SARS-CoV-2 comprising a lactic acid bacterium as an active ingredient.

2. The agent according to claim 1, wherein the lactic acid bacterium is capable of activating plasmacytoid dendritic cells (pDC) and inducing at least interferon (IFN) production.

3. The agent according to claim 1 or 2, wherein the lactic acid bacterium is *Lactococcus lactis* subsp. *lactis.*

4. The agent according to claim 1 or 2, wherein the lactic acid bacterium is *Lactococcus lactis* subsp. *lactis* JCM 5805.

5. The agent according to claim 1 or 2, which is an agent for preventing onset of COVID-19.

6. The agent according to claim 1 or 2, which is in the form of a food composition.

7. The agent according to claim 6, wherein the food composition is a beverage or supplement.

8. The agent according to claim 1 or 2, which is in the form of a pharmaceutical composition.

9. Use of a lactic acid bacterium, for the manufacture of an agent for inhibiting proliferation of SARS-CoV-2, an agent for preventing onset of COVID-19 or an agent for relieving a symptom of COVID-19, as an agent for inhibiting proliferation of SARS-CoV-2, an agent for preventing onset of COVID-19 or an agent for relieving a symptom of COVID-19, or in a method for inhibiting proliferation of SARS-CoV-2, a method for preventing onset of COVID-19 or a method for relieving a symptom of COVID-19.

10. A lactic acid bacterium or a composition comprising the lactic acid bacterium, for use in inhibiting proliferation of SARS-CoV-2, preventing onset of COVID-19 or relieving a symptom of COVID-19.

11. A method for inhibiting proliferation of SARS-CoV-2, a method for preventing onset of COVID-19 or a method for relieving a symptom of COVID-19, which method comprises a step of feeding or administering an effective amount of a lactic acid bacterium or a composition comprising the lactic acid bacterium to a subject in need thereof.
